# EUROPEAN PATENT APPLICATION

(11) **EP 1 880 992 A1**
(43) Date of publication of application: **23.01.2008**
(21) Application number: 07117155.7
(22) Date of filing: 23.07.2004
(51) Int. Cl.: C07D 209/14, A61K 31/404

(54) **Stable modifications of tegaserod hydrogen maleate**

(30) Priority: 24.07.2003 US 489638 P
(62) Divisional of application: 04763452.2
(71) Applicant: Novartis AG, 4056 Basel (CH); Novartis Pharma GmbH, 1235 Vienna (AT)
(72) Inventor: Pfeffer, Sabine, 79576, Weil am Rhein (DE); Vitzling, Christian, 75012, Paris (FR)
(74) Representative: Barbier, Denis Robert

(57) **Abstract**

The present invention relates to crystalline forms of tegaserod hydrogen maleate, their use in pharmaceutical compositions useful in the treatment of irritable bowel syndrome, gastro-esophageal reflux disease, functional dyspepsia, chronic constipation or diarrhea, and their production.

## Description

This invention relates to a stable modification of the partial 5-HT₄ receptor 3-(5-methoxy-1 H-indol-3-yl-methylene)-N-pentylcarbazimidamide hydrogen maleate, herein referred to as tegaserod hydrogen maleate (I):

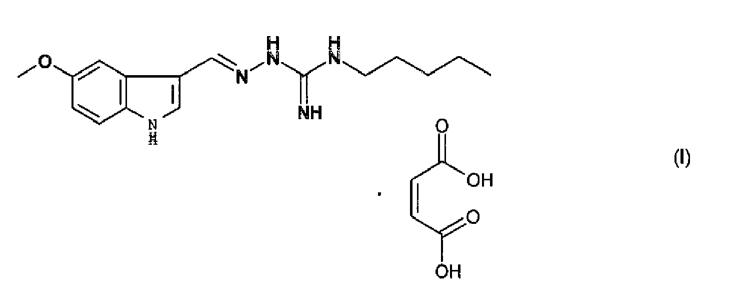

In addition, the invention relates to pharmaceutical compositions comprising the modification of the invention, to uses of said modification in medicine and to processes to obtain them. Such pharmaceutical compositions are particularly relevant to the treatment of conditions for which a partial agonist of 5-HT₄ receptors is required, such as gastrointestinal disorders (GI disorders) which affect the gastrointestinal tract from the mouth to the anus. Such GI disorders may be but are not limited to, heartburn, bloating, postoperative ileus, abdominal pain and discomfort, early satiety, epigastric pain, nausea, vomiting, burbulence, regurgitation, intestinal pseudoobstruction, anal incontinence, irritable bowel syndrome (IBS), gastro-esophageal reflux disease (GERD), functional dyspepsia, chronic constipation or diarrhea, gastroparesis, e.g. diabetic gastroparesis, ulcerative colitis, Crohn's disease, ulcers, gastrointestinal hyperactivity, and relaxing effect on intestinal smooth muscle cells and the visceral pain associated therewith.

European patent 0 505 322 describes a family of aminoguanidines derivatives including tegaserod and pharmaceutically acceptable salts thereof as 5-HT₄ receptor agonists. The pharmaceutically acceptable salts include acid addition salts, specifically the hydrochloride, hydrobromide, hydrofluoride, sulphate or bisulphate, phosphate or hydrogen phosphate, acetate, besylate, citrate, fumarate, gluconate, lactate, maleate, malonate, malate, mesylate, succinate and tartrate salts. A preferred salt form for medical use is the hydrogen maleate salt.

It has been found that the substance tegaserod hydrogen maleate can exist in more than one modification or solvate (crystal form and modification are herein used interchangeably). It is thus an object of the present invention to provide the two identified modifications of tegaserod hydrogen maleate, referred herein as to modifications A and B, and the 3 identified solvates, referred herein as acetone, isopropanol and ethanol solvates. In particular, modification A is provided with advantageous properties over the other crystalline form B and the solvates. Another object of the present invention is to provide a process for the preparation of modification A, substantially free from other forms of tegaserod hydrogen maleate. Additionally, it is an object of the present invention to provide pharmaceutical formulations comprising modification A of tegaserod hydrogen maleate. Furthermore, crystallization experiments with ethanol, acetone, isopropanol, methanol and with a 1:1 mixture of acetone/hexane causes the formation of solvates.

Crystal modifications A and B and the solvates of tegaserod hydrogen maleate differ with respect to their thermodynamic stability, in their physical parameters, such as the absorption pattern in an Infrared Absorption Spectrometry (IR), the phase transition signal in Differential Scanning Calorimetry (DSC), the powder diffraction pattern in X-ray and in their preparation processes.

Modification A is a crystal form exhibiting advantageous properties, such as being well-defined, being more stable when exposed to heat and more stable in the presence of water. Modification B can under certain conditions, e.g. heat or water containing organic solvents, completely or partly be converted into modification A. Thus, modification A is thereby characterized in being thermodynamically more stable than modification B. Similarly the solvates, e.g. the solvate with acetone can be transformed into modification A by equilibration with water.

The invention relates to the crystal modification A, to its preparation, and its use in pharmaceutical preparations. Preferably the invention relates comprising the crystal modification A of greater than 90%, more preferably 95%, more preferably 96%, more preferably 97%, more preferably 98%, more preferably 99% polymorphic purity as determined, e.g. by X-ray powder diffraction, Raman spectroscopy and IR spectrum. The invention relates preferably to the essentially pure form of the modification A of tegaserod hydrogen maleate. The term "essentially pure" as used herein means that less than about 5% and preferably less than about 2% by weight in the preparation, e.g. as a pure drug substance or in a pharmaceutical composition is not the modification A of tegaserod hydrogen maleate (but another form of tegaserod hydrogen maleate, e.g. modification B or any of the solvates), based upon 100% total weight of the tegaserod hydrogen maleate in the preparation, e.g. as pure drug substance or in a pharmaceutical composition.

The crystal modification A of tegaserod hydrogen maleate has a melting signal at about 190°C measured by DSC. In the infrared (IR) spectrum, modification A differs from modification B predominantly in the shape and in the relative intensity of many bands in the areas 3000 - 4000 cm⁻¹, around 1150 cm⁻¹ and 800 - 1000 cm⁻¹. Particularly characteristic is the sharp band at 3415 cm⁻¹, which is not present in the IR spectrum of the modification A, but in the spectra of modification B. In the range 4000-600 cm⁻¹, inter alia the following bands are obtained for modification A [cm⁻¹]: 3410-3100 (v N-H/ v (N-H)⁺); 2990-2872 (ν_{asy} / v_{sy} of aliphatic CH₂/CH₃ νNH₂⁺); 2830 (v CH of OCH₃); 1710 (sh) (v C=0 of RCOOH (hml)); 1669/1629/1583 (v C=N /ν C=C of indole include. ν C=O (R-COO⁻) and δ-NH); 1488 (v C=O (R-COO-) / δₐₛ (CH) aliphatic); 1150-1028 (v CO); 860-810 (δ-CH o.o.p. aromatic). For example, the apparatus FT IR 9725-X (Perkin Elmer) can be used for recording of each of the IR spectra.

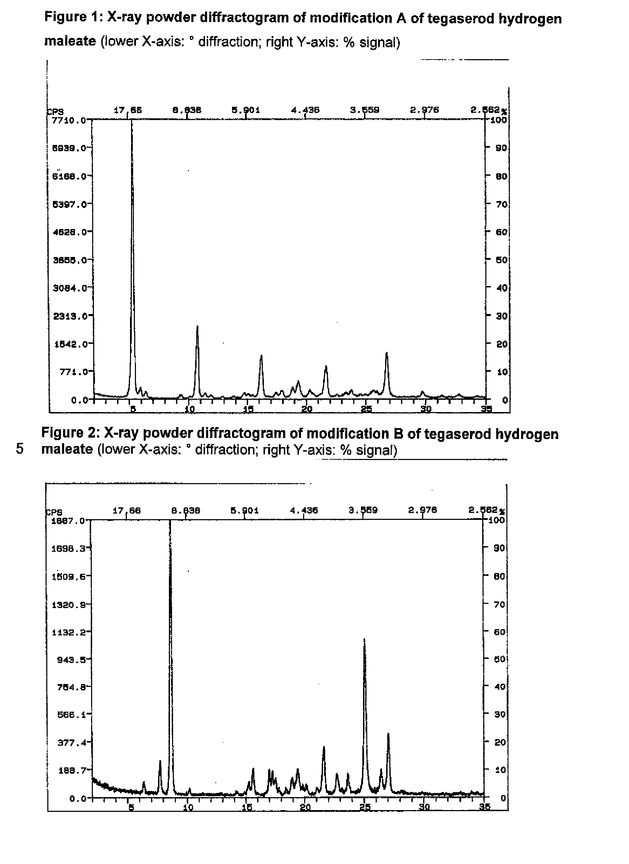

The modification A has an X-ray powder diffraction pattern with characteristic peaks. Modification A differs from modification B by the presence of significant peaks (see below Table 1). All characteristic peaks are summarized in Table 1 and shown in Fig. 1. The measurement is carried out, for example, on a diffractometer such as Scintag 2000 (Santa Clara, USA) using copper Ka radiation (45 KV, 40 mA).

**Table 1: X-ray powder diffraction pattern:**

| **Modification** | **List of significant peaks** |
|---|---|
| Modification A comprising the following peaks | 5.4°, 5.9°, 6.4°, 10.8°, 16.2°, 19.3°, 21.7°, 26.8° (all values ± 0.3°) |
| Modification B comprising the following peaks | 7.7°, 8.7°, 21.6°, 25.1°, 27.0° (all values ± 0.3°) |

Characteristic for the modification A is the thermogram in differential scanning calorimetry (DSC). It has an endothermic melting peak at about 190°C. The melting enthalpy of the endothermic signal is 130 J/g ±5 J/g. The measurement is carried out on a Perkin Elmer DSC 7 in a crimped pan with a heating rate of 10 K/minute. The typical sample quantity is about 2 mg. As a typical distinguishing feature compared with the modification B and the acetone solvate, ethanol solvate, the thermogram of the modification A has no further thermal signal (see Table 1 a below): Table 1a: The DSC data of modifications A and B, as well as the solvates are as follows:

| | |
|---|---|
| Modification A | ΔH = 130 J/g, peak 190°C |
| Modification B | peak 191 °C, additional peaks at 97°C and 181 °C |
| Acetone solvate | peak 191°C, additional peaks at 127°C and 182°C |
| Ethanol solvate | peak 191°C, additional peaks at 145°C and 182°C |

The modification B has an X-ray powder pattern with different characteristic diffraction peaks in comparison to modification A. It differs from the modification A in the X-ray powder pattern in that it shows a characteristic peak at 8.7°. The IR spectrum of modification B shows a different pattern in the areas of 3415 cm⁻¹ (sharp, single signal), 1150 cm⁻¹ (less intense signal) and 800-1000 cm⁻¹ (more intense signals) in comparison to modification A. Characteristic for the modification B is also the thermogram in the differential scanning calorimetry measurement. It has an endothermic peak in addition to the one at about 190°C, at 97°C and melting/crystallization signals at about 181 °C. It is found that modification B converts into modification A when heating is applied (thus the signal at about 190°C). The measurement is done as described above.

The modification A has significant advantages compared to the modification B and the ethanol, acetone solvate.

Thus, it was found, for example, that modification A is more stable when exposed to heat and more stable in the presence of water. Both properties of modification A have e.g. advantages for tablet manufacturing. E.g. the drying procedure in the tablet manufacturing can be done with higher temperature. Furthermore, the fact that modification A is more stable in the presence of water means that the wet granulation technique can be used in the manufacture of tegaserod and the tablets comprising modification A shows a better stability to moisture. This is advantageous as due to poor solubility of tegaserod hydrogen maleate, a formulation was developed (see WO 00/10526) which used a high amount of hygroscopic disintegrants (>15%). This high amount of disintegrant attract water within the tablet and would if the particular modification is not stable in the presence of water require particular packaging devices. Furthermore, it is found surprisingly that the process comprising certain amount of water and organic solvent in the crystallization or re-crystallization step of tegaserod hydrogen maleate is advantageous to the one known in the prior art EP 0 505 322.

The invention relates to the modification A of tegaserod hydrogen maleate, consisting of a characteristic crystal structure according to Table 2, determined by means of an X-ray single crystal analysis.

**Table 2: Crystal Data and Refinement Details**

| crystal system | triclinic |
|---|---|
| space group | P-1 |
| a, Å | 8.640 |
| b, Å | 15.800 |
| c, Å | 17.572 |
| α, Å | 68.67 |
| β, Å | 88.10 |
| γ, Å | 88.02 |
| V, Å³ | 2232 |
| Z | 4 |
| D(calc), g/cm³ | 1.242 |

The invention relates to the modification A of tegaserod hydrogen maleate, having the characteristic X-ray powder diffraction pattern as shown in Table 1 or Fig. 1.

The invention relates to the modification A of tegaserod hydrogen maleate, having in the thermogram in differential scanning calorimetry a single melting signal at about 190°C. The melting enthalpy of the endothermic signal is about 130 J/g.

The invention relates to the modification A of tegaserod hydrogen maleate, lacking the following absorption in the infrared spectrum (KBr pellet--transmission method): sharp, single band at 3415 cm⁻¹.

According to the invention there is further provided a process for the preparation of the modification A of tegaserod hydrogen maleate.

Modification A of tegaserod hydrogen maleate may be prepared by crystallization or recrystallization of any form, or mixtures of any forms of tegaserod hydrogen maleate in a solution comprising e.g. water and appropriate solvent.

A preferred embodiment of the invention relates to a process for the preparation of modification A of tegaserod hydrogen maleate, comprising the step of crystallizing any form, or mixtures of any forms of tegaserod hydrogen maleate in a solution comprising an organic solvent and water.

A more preferred embodiment of the invention relates to a process for the preparation of modification A of tegaserod hydrogen maleate, comprising the step of crystallizing any form, or mixtures of any forms of tegaserod hydrogen maleate in a solution comprising water and acetate ester; e.g. n-butyl acetate, isobutyl acetate or ethyl acetate and water; preferably ethyl acetate and water.

A more preferred embodiment of the invention relates to processes for the preparation of modification A of tegaserod hydrogen maleate as described above, wherein between 0.01 and 5 weight % water of the total weight of said water-organic solvent solution is present, more preferably between 0.1 and 4%, more preferably between 1 and 3 %. Most preferably, the water is present in an amount in which the water is just soluble in said organic solvent, e.g. in about 2.8 weight % water of the total weight of the water - ethyl acetate solution.

Furthermore, the crystallization may be e.g. done at temperature between about 10°C up to the boiling temperature of said water-organic solvent solution, e.g. for the 2.8 weight % water-ethyl acetate solution between 10°C and 75°C, preferably around 70°C.

Working up the crystallized modification A of tegaserod hydrogen maleate and purification thereof thus obtained may be carried out in accordance to known procedures.

The invention also relates to modification B of tegaserod hydrogen maleate comprising the characteristic X-ray powder diffraction pattern as shown in Table 1, preferably having the characteristic X-ray powder diffraction pattern as shown in Table 1.

The invention relates to the modification B of tegaserod hydrogen maleate, having in the thermogram in differential scanning calorimetry next to the melting signal at 190°C (as for modification A), one additional endothermic peak at 97°C (very weak) and melting/crystallization signals at about 180°C (weak).

The invention relates to the modification B of tegaserod hydrogen maleate, having the following absorption in the infrared spectrum (KBr pellet--transmission method): sharp, single band at 3415 cm⁻¹.

The invention relates to pharmaceutical preparations comprising the modifications A or B (or mixtures of A and B) of tegaserod hydrogen maleate. The invention relates in particular to corresponding pharmaceutical preparations for the treatment of irritable bowel syndrome, gastro-esophageal reflux disease, functional dyspepsia, chronic constipation or diarrhea and subindications thereof. The invention relates to the use of the modifications A or B of tegaserod hydrogen maleate for the preparation of pharmaceutical preparations, in particular for the treatment of irritable bowel syndrome, gastro-esophageal reflux disease, functional dyspepsia, chronic constipation or diarrhea and subindications thereof.

The modifications A or B (or mixtures of A and B) of tegaserod hydrogen maleate can be used, for example, in the form of pharmaceutical preparations which comprise a therapeutically effective amount of the active ingredient, if desired together with inorganic or organic, solid or liquid, pharmaceutically usable carriers, which are suitable for enteral, for example oral, or parenteral administration. Furthermore, the modifications A and B of tegaserod hydrogen maleate can be used in the form of preparations which can be administered parenterally or of infusion solutions. The pharmaceutical preparations may be sterilized and/or may comprise excipients, for example preservatives, stabilizers, wetting agents and/or emulsifiers, solubilizers, salts for regulating the osmotic pressure and/or buffers. The present pharmaceutical preparations comprise from about 0.1 % to 100%, in particular from about 1% to about 50%, of lyophilisates to about 100% of the active ingredient.

The invention also relates to the use of the modifications A and B of tegaserod hydrogen maleate as a drug, preferably in the form of pharmaceutical preparations. The dosage may depend on various factors, such as method of administration, species, age and/or individual condition. The doses to be administered daily are between about 0.0016 and about 16 mg/kg in the case of oral administration, and preferably between about 1.2 mg and about 120 mg, more preferably about 12 mg, e.g. twice daily a 6 mg for warm-blooded species having a body weight of about 70 kg.

### Brief description of the Figures:

Fig. 1 is an X-ray powder diffractogram of modification A of tegaserod hydrogen maleate (lower X-axis: ° diffraction; right Y-axis: % signal)
Fig. 2 is an X-ray powder diffractogram of modification B of tegaserod hydrogen maleate (lower X-axis: ° diffraction; right Y-axis: % signal)

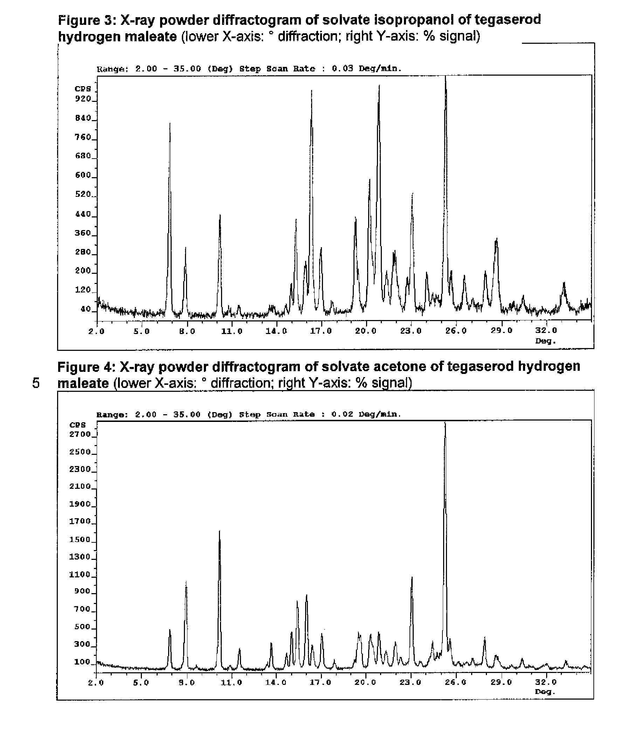

Fig. 3 is an X-ray powder diffractogram of solvate isopropanol of tegaserod hydrogen maleate (lower X-axis: ° diffraction; right Y-axis: % signal)

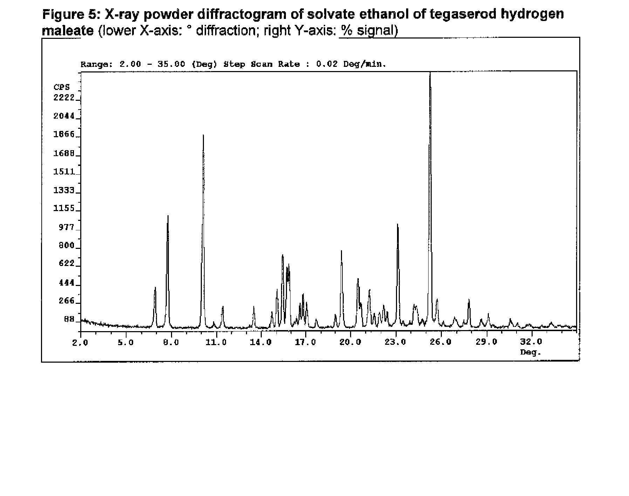

Fig. 4 is an X-ray powder diffractogram of solvate acetone of tegaserod hydrogen maleate (lower X-axis: ° diffraction; right Y-axis: % signal)
Fig. 5 is an X-ray powder diffractogram of solvate ethanol af tegaserod hydrogen maleate (lower X-axis: ° diffraction; right Y-axis: % signal)

The preparation of the modifications A, mixtures of A + B, modification B and the solvate isopropanol, solvate acetone, solvate ethanol is carried out, for example, as described in the embodiments below.

### EXAMPLES:

### EXAMPLE 1: Preparation of 3-(6-methoxy-1H-indol-3-yl-methylene)-N-pentylcarbazimidamide

### Preparation of S-Dodecylisothiosemicarbazide hydrobromide

Charge a suitable reactor with approximately 860 L of ethanol anhydrous.
Then, while stirring, add 96 kg (1056 moles) of thiosemicarbazide and 306 kg (1210 moles) of 1-bromododecane.
Heat the reaction mixture to 68-70°C and stir it at this temperature for 15 to 20 hours. Verify the completion of the reaction by TLC (requirement: < 0.1% m/V thiosemicarbazide in the reaction mixture). In case result does not meet the requirement, continue the reaction and repeat the TLC test at 4 hours interval.
Then dilute the mixture with approximately 384 L of ethanol anhydrous and cool it to 38-42 °C.
Cool the mixture gradually to approximately -10°C. Stir it at this temperature for at least 2 hours. Transfer the slurry to a filter and collect the solids.
Wash the product with several portions of cold ethanol anhydrous. Discard filtrate and wash solution.
Dry the product (*S-Dodecylisothiosemicarbazide hydrobromide*) at a temperature not exceeding 50°C under reduced pressure.

### Preparation of 3-(5-Methoxy-1H-indol-3-ylmethylene)-N-pentylcarbazimidamide hydrobromide

Charge a suitable reactor with approximately 340 L of ethanol anhydrous. While stirring, add:
- 63.3 kg (186 moles) of S-Dodecylisothiosemicarbazide hydrobromide, and
- 24.8 kg (284 moles) of 1-pentylamine.

Heat the reaction mixture to 60-64°C and stir it at this temperature for at least 6 hours. Verify the completion of the reaction by TLC (requirement: < 0.1 % mN S-Dodecylisothiosemicarbazide hydrobromide in the reaction mixture). In case result does not meet the requirement, continue the reaction and repeat the TLC test at 4 hours interval. Then cool the reaction mixture to 20-25°C.

Under reduced pressure, while stirring, add approximately 5 kg of hydrogen chloride (gas) to obtain a pH between 1 and 4. Check pH.
Add in several portions 30.1 kg (172 moles) of 5-methoxyindol-3-carbaldehyde.
In between the addition the reaction mixture may be seeded with crystals of 3-(5-Methoxy-1H-indol-3-ylmethylene)-N-pentylcarbazimidamide hydrobromide.
Then add 35 L of ethanol anhydrous and check the pH again (requirement: between 1 and 4; otherwise introduce more hydrogen chloride gas). Stir the reaction mixture for about 1 hour.
Then heat the mixture to 38-42°C and add approximately 395 L of tert-butyl methyl ether.
Then cool the mixture to 0-5 °C and stir it at this temperature for about 1.5 hours.
Then transfer the slurry to a filter and collect the solids. Rinse vessel and connecting lines and wash the cake in several portions with sufficient amounts of cold tert-butyl methyl ether/ethanol anhydrous 3:1 (v/v).
Discard filtrate and wash solution and dry the product at a temperature not exceeding 60°C under reduced pressure.

### Preparation of 3-(5-methoxy-1H-indol-3-yl-mathylene)-N-pentylcarbazimidamide

Charge suitable vessel with approximately 1045 L of isopropyl acetate and 114 L of methanol.
While stirring, add:
135.6 kg (354 moles) of 3-(5-Methoxy-1 H-indol-3-ylmethylene)-N-pentylcarbazimidamide hydrobromide.
Cool the mixture to 15-20°C and while keeping this temperature add a mixture of approximately 120 L of water and 93 kg of sodium hydroxide, 30% aqueous solution. Stir the reaction mixture until all solids are dissolved, then transfer the mixture to a separator and allow the phases to separate. Discard the aqueous phase. As an option, the extraction of the organic phase with water/sodium hydroxide, 30% aqueous solution can be repeated.
Transfer the organic phase to another vessel. Wash the first vessel, the separator and supply lines with a sufficient amount of isopropyl acetate/methanol 9:1 (v/).
Combine the organic phase with the wash and concentrate under reduce pressure to a volume of about 330 L.
Cool the concentrate to 25-30°C, and if necessary seed the solution with crystals of 3-(5-methoxy-1H-indol-3-yl-methylene)-N-pentylcarbazimidamide.

Then cool to 0-2 °C and stir at this temperature for at least 1 hour.
Transfer the slurry to a filter and collect the solids. Rinse the vessel and connecting lines and wash the cake in several portions with sufficient amounts of cold isopropyl acetate.
Then in addition wash the cake in several portions with a sufficient amount of water. Discard filtrate and wash solution and dry 3-(5-methoxy-1H-indol-3-yl-methylene)-N-pentylcarbazimidamide at a temperature not exceeding 70°C under reduced pressure.

### EXAMPLE 2: Preparation of modification A of the maleate salt of 3-(6-methoxy-1H-indol-3-yl-methylene)-N-pentylcarbazimidamide

60.3 g of 3-(5-methoxy-1H-indol-3-yl-methylene)-N-pentylcarbazimidamide is dissolved in a mixture of 1167 g ethyl acetate and saturated with water, i.e. 33g (i.e. 2.8 weight % water based on the total weight of the mixture; mixture = crystallization solution) at 60-65° C while stirring. The obtained solution is cooled to 50-55 °C and 1.2 g activated carbon is added. The solution is filtered and after which washing with 120 g ethyl acetate/water (2.8% weight water based on the total weight of the mixture) is carried out. Then a solution A containing 23.9 g maleic acid and 35.8 g water is added at 50-55 °C over a period of 20-30 minutes. A suspension forms. The suspension is stirred for 15-30 minutes at 50-55 °C and then stirred for 2 hours at 68-70°C. Afterwards the suspension is cooled to 20-25°C during a period of 1.5 to 2 hours. The product (= modification A of the hydrogen maleate salt of 3-(5-methoxy-1H-indol-3-yl-methylene)-N-pentylcarbazimidamide) is isolated by filtration and washed with 120 g ethyl acetate. By drying in vacuo at 55-60° C for 18 hours, the product is obtained in a yield of about 80.8 g.

### EXAMPLE 3: Preparation of a mixture of modification A and modification B, modification B, acetone solvate, and ethanol solvate of the maleate salt of 3-(5-methoxy-1H-indol-3-yl-methylene)-N-pentylcarbazimidamide,

### Mixture of modification A and B:

30.0 g of 3-(5-methoxy-1H-indol-3-yl-methylene)-N-pentylcarbazimidamide is dissolved in 450 ml acetone at 20-30°C. 0.6 g activated carbon is added and the suspension is stirred for 30 minutes. The suspension is filtered and washed with 30 ml acetone. The filtrate is heated to 50-54°C and 11.55 g maleic acid in 80 ml acetone during 15 minutes is added. A suspension forms which is stirred for another 15 minutes. The suspension is cooled to 0-5°C and stirred for 2 hours at this temperature. The product (= mixture of modification A and modification B of the hydrogen maleate salt of 3-(5-methoxy-1 H-indol-3-yl-methylene)-N-pentylcarbazimidamide) is isolated by filtration and washed with 70 ml acetone at 0-5°C. By drying in vacuo at 80° C, the product is obtained in a yield of about 39.6 g.

### Modification B

The product obtained above is crystallized from tetrahydrofuran and methanol. Then it is recrystallized with ethanol and ether at 90°C.

### Solvate acetone

The product obtained above is crystallized at 60°C by fast crystallization (e.g. evaporation) from hot saturated solution of acetone. Dried in air.

### Solvate ethanol

The product obtained above is crystallized at 60°C by fast crystallization (e.g. evaporation) from hot saturated solution of ethanol. Dried in air.

### Solvate isopropanol

The product obtained above is crystallized at 60°C by fast crystallization (e.g. evaporation) from hot saturated solution of isopropanol. Dried in air.

### Example 4: A 2 mg tablet formulation of modification A may be prepared as described hereinafter

### a) Preparation of the granulated material:

### Premixing step:

1. 4.432 kg of modification A of the hydrogen maleate salt of 3-(5-methoxy-1H-indol-3-yl-methylene)-N-pentylcarbazimidamide and 28.688 kg of lactose monohydrate are mixed with an intensive mixer (Colette Gral® 300 1 or Fielder® ; mixer speed setting : 1; chopper speed setting : 1) for approximately 1.5 minutes, or with a free fall mixer (Turbula®, Soneco® or Röhnrad®)
2. The pre-mixture from step 1 is then sieved (oscillating granulator, e. g., Frewitt® or Erweka®; mesh size: 0.8 millimetres).
3. The pre-mixture is divided into two portions of 16.560 kg.

Preparation of the granulating solution
4. Approximately 40 kg of purified water are weighed out.
5. 3.600 kg of methylhydroxypropylcellulose 3 maps are added to the purified water from step 4 and this is stirred until dissolution.
6. 1.440 kg of poloxamer 188 are added to the solution from step 5 while stirring until dissolution.

Granulating step:
7. 28.800 kg of crospovidone, 10.080 kg of lactose monohydrate and 4.320 kg of glyceryl monostearate are weighed out.
8. One portion of the premixture from step 3 is added to the excipients from step 7 and this is mixed with the intensive mixer, e.g., Colette Gral® 300 I or Fielder® (mixer speed setting : 1; chopper speed setting : 1) for approximately 2 minutes.
9. The mixture from step 8 is wetted with the granulating solution from step 6 while mixing with the intensive mixer, e.g., Colette Gral® 300 I or Fielder® (mixer speed setting : 1; chopper speed setting : 0; pumping rate approximately : 4 kg/minute) for approximately 12 minutes.
10. Approximately 2 kg of purified water are weighed out.
11. The vessel from step 6 is rinsed with the purified water from step 10 and this is added to the mixture from step 9 while mixing.
12. The mass is granulated by mixing with the intensive mixer, Colette Gral® 300 I or Fielder® (mixer speed setting : 1; chopper speed setting : 1) for approximately 2.5 minutes.

Drying step
13. The granulate from step 12 is dried in a fluidised air bed drier (e. g., Glatt® or Aeromatic®) for approximately 65 minutes (inlet air temperature approximately 70°C) to reach the required loss on drying (LOD) for the tabletting mixture, i. e., until LOD - <4,4%.
14. The granulate sized by sieving (0.8 millimetres) with an oscillating sieve granulator, e.g., Frewitt® or Erweka®.
15. Steps 4 to 14 are repeated with the other portion of step 3.

### b) Preparation of the tabletting mixture

16. 8.640 kg of polyethylene glycol 4000 and 5.760 kg of glyceryl monostearate are sieved (oscillating granulator, e.g., Frewitt® or Erweka®; mesh size: 0.8 millimetres)
17. The ingredients from step 16 are added to the total mass of granulated material and this is mixed with a free fall mixer, e.g., Saneco® or Röhnrad®, for approximately 20 minutes (10 rpm) to obtain the desired final tabletting mixture.

c) Compression step
18. The tabletting mixture from step 17 is pressed into tablets using compression pressures of 11, 14 or 17 KN on a rotary tabletting machine, e.g., Fette®, Korsh®, Kefian® or Coarty® (temperature < 20°C; M (relative humidity) < 40%)

### Example 5: Composition of a 2 mg tablet (1 mg of base corresponds to 1. 385 mg of modification A of the hydrogen maleate salt of 3-(5-methoxy-1H-indol-3-yl-methylene)-N-pentylcarbazimidamide)

| | |
|---|---|
| Modification A of the hydrogen maleate salt of 3-(5-meth oxy-1H-indol-3-yl-methylene)-N-pentylcarbazimidamide | 2.77 mg (2mg base) |
| Polyplasdone XL USP/NF | 36.00 mg |
| Glyceryl monostearate USP/NF | 9.00 mg |
| Poloxalkol | 1.80 mg |
| Lactose 200 mesh | 30.53 mg |
| HPMC 3cPs | 4.50 mg |
| Polyethyleneglycol 4000 | 5.40 mg |
| Water adsorbed | 2.00 mg |
| Total | 92 mg |

### Example 6: A 6 mg tablet formulation of modification A may be prepared by the manufacturing process described hereinafter.

a) Preparation of the granulated material
   Preparation of the granulating solution
   1. Approximately 40 kg of purified water are weighed out.
   2. 3.600 kg of methylhydroxypropylcellulose 3 maps are added to the purified water from step 1 while stirring until dissolution.
   3. 1.440 kg of poloxamer 188 are added to the solution from step 2 while stirring until dissolution (mixing tank under stirring).
   Granulating step
   4. 4.787 kg of modification A of the hydrogen maleate salt of 3-(5-meth oxy-1 H-indol-3-yl-methylene)-N-pentylcarbazimidamide and 28.800 kg of crospovidone, 21.853 kg of lactose monohydrate and 4.320 kg of glyceryl monostearate are weighed out.
   5. The ingredients from step 4 are mixed with the intensive mixer, e.g., Colette Gral® 300 l or Fielder® (mixer speed setting: 1; chopper speed setting : 1) for approximately 2 minutes.
   6. The mixture from step 5 is wetted with the granulating solution from step 3 while mixing with the intensive mixer, e.g., Colette Gral® 300 l or Fielder® (mixer speed setting: 1; chopper speed setting : 0; pumping rate approximately 4 kg/minute) for approximately 12 minutes.
   7. Approximately 2 kg of purified water are weighed out.
   8. The vessel from step 3 is rinsed with the purified water from step 7 and this is added to the mixture from step 6 while mixing.
   9. The mass is granulated by mixing with the intensive mixer, e.g., Colette Gral® 300 l or Fielder® (mixer speed setting: 1; chopper speed setting : 1) for approximately 2.5 minutes.
   Drying step
   10. The granulate from step 9 is dried in a fluidised air bed drier, e.g. , Glatt® or Aeromatic® for approximately 65 minutes (Inlet air temperature approximately 70°C) to reach the desired loss on drying (LOD) for the tabletting mixture, i. e., until LOD ≤4,4%.
   11. The granulate sized by sieving (0.8 millimetres) with an oscillating sieve granulator (Frewitt® or Erweka®
   12. Steps 1 to 11 are repeated.
b) Preparation of the tabletting mixture
   13. 8.640 kg of polyethylene glycol 4000 and 5.760 kg of glyceryl monostearate are sieved with an oscillating sieve granulator, e.g., Frewitt® or Erweka® (0.8 millimetres)
   14. The ingredients from step 13 are added to the total mass of granulated material and this is mixed with a free fall mixer, e.g., Soneco® or Röhnrad®, for approximately 20 minutes (10 rpm) in the desired final tabletting mixture.
c) Compression step
   15. The tabletting mixture from step 14 is pressed into tablets using compression pressures of 13, 16 or 19 KN on a rotary tabletting machine, e.g., Fette®, Korsh®, Kelian® or Coarly® (temperature<200C, M (relative humidity) < 40 %).

### Example 7: Composition of a 6 mg tablet (1 mg of base corresponds to 1. 385 mg of modification A of the hydrogen maleate salt of 3-(5-methoxy-1H-indol-3-yl-methylene)-N-pentylcarbazimidamide)

| | |
|---|---|
| Modification A of the hydrogen maleate salt of 3-(5-meth oxy-1 H-indol-3-yi-methylene)-N-pentylcarbazimidamide | 8.31 mg (6mg base) |
| Polyplasdone XL USP/NF | 50.00 mg |
| Glyceryl monostearate USP/NF | 12.50 mg |
| Poloxalkol | 2.50 mg |
| Lactose 200 mesh | 37.94 mg |
| HPMC 3cPs | 6.25 mg |
| Polyethyleneglycol 4000 | 7.50 mg |
| Water adsorbed | 3.00 mg |
| Total | 128 mg |

### EXAMPLE 8: Formulations of modification A of the maleate salt of 3-(5-methoxy-1H-indol-3-yl-methylene)-N-pentylcarbazimidamide

A 6 mg tablet is prepared using roller compaction (see also Example 4 of WO 03/053432):

| Component | Quantity |
|---|---|
| | (125 mg tablet) % w/w |
| Modification A of Tegaserod hydrogen maleate | 6.65 |
| Lactose spray dried | 76.85 |
| Crospovidone | 10.00 |
| Aérosil | 0.50 |
| glyceryl behenate | 6.00 |

Compositions are prepared by mixing tegaserod maleate, lactose, crospovidone, aérosil and glyceryl behenate. This mixture is compacted by roller compaction and milled. Tablets are formed by compression.

### EXAMPLE 9: Further compositions (direct compression)

Further compositions may be obtained by
(i) preparing a mixture of tegaserod maleate (prepared as above), diluent and lubricant,
(ii) sieving the mixture
(iii) adding the disintegrant, glidant, lubricant and optionally binder and blending the sieved mixture of step (ii) and
(iv) forming tablets by direct compression.

Part of the lubricant may be added in the mixture of step (i), the rest in the final mixture of step (iii) or the total amount of lubricant may be added in the final mixture of step (iii).

The resulting powder blends of step iii) are compressed on either a single punch press (Korsh EKO), 6 station-rotary press (Korsh PH106), 17 station-rotary press (Korsh PH 230) or 43 station-rotary press (Fette PT2090).

All components may be mixed together, sieved through and mixed again. Tablets are then formed by direct compression.

A 6 mg tablet is prepared using the direct compression method.

| Component | Quantitiy (125 mg tablet) %w/w |
|---|---|
| Modification A of the hydrogen maleate salt of 3-(5-meth oxy-1H-indol-3-yl-methylene)-N-pentylcarbazimidamide | 6.65 |
| Lactose spray dried | 72.25 |
| hydroxy propylmethyl cellulose | 5 |
| Crospovidone | 10.00 |
| Aérosil | 0.10 |
| glyceryl behenate | 6.00 |

A preblend is formed by mixing tegaserod maleate, hydroxy propylmethyl cellulose, a part of glyceryl behenate and a part of lactose. This preblend is mixed with the remaining excipients except glyceryl behenate.This blend is lubricated with the remaining part of glyceryl behenate.
The final blend is compressed using a rotary press (Korsh PH 343 or Fette PT2090) equipped with 7 mm, round upper punches.

## Claims

1. A crystal modification B of tegaserod hydrogen maleate, which has an X-ray powder diffraction pattern comprising the following characteristic peak positions as 2 ⊖ values: 7.7±0.3°, 8.7±0.3°, 21.6±0.3°, 25.1±0.3°, 27.0±0.3°.

2. A crystal modification B of tegaserod hydrogen maleate, having in the thermogram in differential scanning calorimetry one thermal signal at about 191°C and additional peaks at 97°C and 181 °C.

3. The use of a modification according to any of claims 1 to 2 for the manufacture of a medicament for the treatment of irritable bowel syndrome, gastro-esophageal reflux disease, functional dyspepsia, chronic constipation or diarrhea.

4. A pharmaceutical composition comprising a pharmaceutically acceptable carrier or diluent and a therapeutically effective amount of crystal modification B of tegaserod hydrogen maleate according to claims 1 to 2.

5. A pharmaceutical composition according to claim 4 which further comprises modifications A of tegaserod hydrogen maleate.
